# EUROPEAN PATENT SPECIFICATION

(11) **EP 2 121 554 B1**
(45) Date of publication and mention of the grant of the patent: **30.04.2014**
(21) Application number: 08734660.7
(22) Date of filing: 19.03.2008
(51) Int. Cl.: C07C 45/74, C07C 49/203

(54) **ALDOL CONDENSATION**
ALDOLKONDENSATION
CONDENSATION D'ALDOL

(30) Priority: 22.03.2007 EP 07005895
(43) Date of publication of application: 25.11.2009
(73) Proprietor: DSM IP Assets B.V., 6411 TE Heerlen (NL)
(72) Inventor: BONRATH, Werner, 79115 Freiburg (DE); SCHÜTZ, Jan, 79540 Lörrach (DE)
(74) Representative: Dux, Roland
(86) International application number: PCT/EP2008/002178
(87) International publication number: WO 2008/113563

(56) References cited:
- WO-A-01/87812
- JP-A- 3 080 941
- MAMORU AI: "Formation of Acrylaldehyde by Vapour-Phase Aldol Condensation I. Basic Oxide Catalysts" BULL. CHEM. SOC. JPN., vol. 64, 1990, pages 1342-1345, XP002488371

## Description

The invention relates to a novel processes for producing ketones, and more specifically, to processes for producing unsaturated ketones using Ca/Na on silica as solid base supported catalysts. This process results in yields and greater selectivity for the target products, while allowing the recycling of the catalyst.

Aldol reactions are important in the production of intermediates needed to synthesize many commercially important products. The addition of ketones and/ or aldehydes to obtain aldols (β-hydroxy ketones) is a well-known reaction. Dehydration of the resulting aldol to obtain an α,β-unsaturated ketone is also known. Subsequent catalytic hydrogenation of the unsaturated ketone may be carried out to obtain the corresponding saturated higher ketone. However, the starting materials, the intermediate β-hydroxy ketones as well as the α,β-unsaturated ketones are known to those skilled in the art to be quite reactive and susceptible to further consecutive, non-selective condensation, cyclization, and Michael-type addition reactions with the starting ketones and aldehydes, as well as themselves and other ketones and aldehyde by-products.

Many methods have been disclosed in the art to perform aldol condensation reactions. These methods include for example homogeneous, base catalysed aldol reactions such as disclosed in WO 2004041764 or CN 1202065. However, homogeneous catalysed aldol reactions have the disadvantage of exhibiting high salt content often resulting in an unwanted contamination of the final product. Heterogeneous catalysed reactions are also disclosed as versatile methods to catalyze aldol reactions. Heterogeneous catalysis is of fundamental importance in the chemical industry because its "simplicity" as the catalyst can be removed via filtration.

Various heterogeneous catalysts are described to catalyze aldol condensation reactions for example by alkali doped solid catalysts (S. Lippert, W. Baumann, K. Thomke, J. Mol. Canal 1991, 69(2), 199-214; J. I. DiCosimo, C. R. Apesteguia, J. Mol. Catal. 1998, 130(1-2), 177-185), by zeolithes and microporous materials (A. G. Panov, J. J. Fripiat, Catal. Lett. 1999, 57(1,2), 25-32; C. Flego, C. Perego, Appl. Catal. A 2000, 192(2), 317-329), by alkali doped silica (W. J. Ji, Y. Chen, Gaodeng Xuexiao Huaxue Xuebao 1997, 18(2), 277-280; J. J. Spivey, M. R. Gogate, J. R. Zoeller, R. D. Bolberg, Ind. Eng. Chem. Res. 1997, 36(11), 4600-4608; A. S. Canning, S. D. Jackson, E. McLeod, E. M. Vass, Appl. Catal. A 2005, 289(1), 59-65), or by layered double hydroxides (M. del Ario, S. Gutierrez, C. Martin, V. Rives, Phys. Chem. 2001, 3(1), 119-126; M. N. Bennani, D. Tichit, F. Figueras, S. Abouarnadasse, J. Chim. Phys. Phys.-Chim. Biol. 1999, 96(3), 498-509; J. C. A. A. Roelofs, A. J. van Dillen, K. P. de Jong, Catal. Today 2000, 60(3-4), 297-303).

The aldol condensation of citral and acetone to ψ-ionone is also a well investigated reaction. WO 2003047748 discloses rare earth oxide on γ-Al₂O₃ supports as catalysts e.g. suitable for the preparation of ψ-ionone. The catalysts are described to show increased space-time yields, the regeneration of the catalysts being possible by treatment with aqueous alkaline solutions. However, a high catalyst loading is necessary and the activity of the catalyst is rather low.

DE 2148610 and DE2150992 disclose the preparation of α,β-unsaturated ketones useful in the preparation of vitamins or odorous substances by liquid phase condensations of ketones with aldehydes over MnO, ZnO, or CdO. However, due to the use of heavy metal oxides, these processes are environmentally not well accepted. Furthermore the selectivity of these processes is rather low.

PL 147748 discloses the preparation of ionones and methylionones by condensation of citral with acetone or methyl ethyl ketone using anion exchanger catalysts. However, the catalyst is not versatile recycled.

M. J. Climent, A. Corma, S. Iborra, A. Velty, J. Mol. Cat. A: Chemical 2002, 182-183, 327-342; S. Abello, F. Medina, D. Tichit, J. Perez-Ramirez, X. Rodriguez, J. E. Sueiras, P. Salagre, Y. Cesteros, Y., Applied Catalysis, A: General 2005, 281(1-2), 191-198; F. Winter, A. J. van Dillen, K. P. de Jong, Chem. Comm. 2005, 31, 3977-3979 dislose the use of hydrotalcites (Al₂O₃:MgO) as heterogeneous catalyst for the preparation of ψ-ionones.

However, the activity of the catalyst decreases after each run and thus, the process is not attractive for industrial scale application.

Thus, despite the amount of methods available so far, there is still a need for a simple, economically attractive, highly selective and environmentally benign method which allows the aldol condensation products to be formed in good yields and purities and permits the catalyst to be recycled by means of a simple industrial process and makes it possible to guarantee constantly stable yields and process conditions with repeated use of the catalyst.

As a result of extensive screening studies, it has surprisingly been found that the object of the invention is achieved by using Ca/Na on silica (SiO₂) as catalyst for carrying out the aldol condensation reaction, especially a catalyst with a surface area <500 m²/g, in particular with a surface area < 200 m²/g, and a metal loading of about 15 to 35 wt.-% Ca and about 20 to 35 wt.-% Na based on the total weight of the catalyst, preferably with a metal loading of about 20 to 30 wt.-% Ca and about 24 to 32 wt.-% Na.

Thus, the invention relates to a process for the preparation of α,β-unsaturated ketones, the process comprising the step of reacting an aldhyde of formula (1) 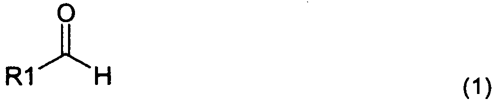 with a ketone of formula (2) 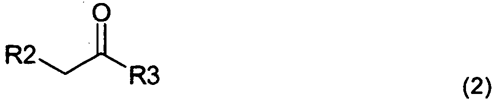 wherein
R1 to R3 represent independently of each other a saturated or unsaturated, branched or non-branched hydrocarbons residue with 1 to 20 C-atoms or a cycloalkyl group with 4 to 12 C-atoms, which optionally may be substituted e.g. by one or several methoxy groups in the presence of Ca/Na on silica aldolization catalyst (in the following referred to as "supported catalyst").

The following scheme illustrates the aldol condensation reaction according to the invention whereby the compound in bracket is passed through as intermediate.

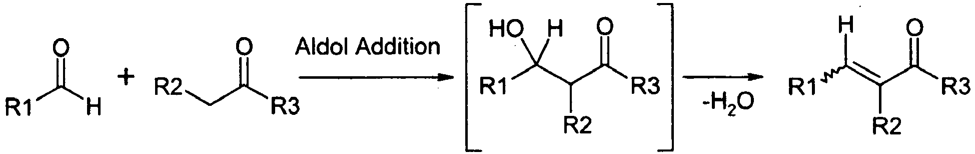

The addition of the ketone to the aldehydes results in an intermediate aldol (β-hydroxy ketones) which is dehydrated in situ to the respective α,β-unsaturated ketone. This reaction is catalysed by the supported catalyst according to the invention.

In a preferred embodiment, the invention relates to a process for the production of α,β-unsaturated ketones of formula (3) 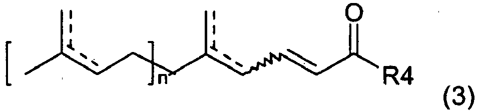 the process comprising reacting a methyl ketone of formula (4) 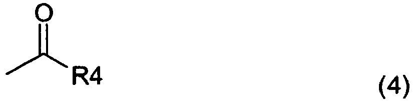 and an aldehyde of formula (5) 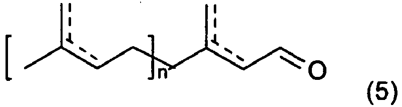 wherein
R4 represents a saturated or unsaturated, linear or branched hydrocarbons residue with 1 to 20 C-atoms, preferably a saturated or unsaturated hydrocarbons residue with 1 to 10 C-atoms, more preferably a saturated hydrocarbons with 1 to 5 C-atoms (i.e. 1,2,3,4,or 5 C-atoms), and
n represents a number from 0 to 4, preferably from 0 to 2 and
wherein the dotted lines indicate, independently of each other, either a saturated or a double bond and if the dotted lines represent a double bond, it can be arranged in one of the two indicated positions and
wherein the process is carried out in the presence of Ca/Na on silica as aldolization catalyst.

In a particular embodiment the invention relates to a process as outlined above wherein R4 represents a saturated linear hydrocarbon with 1 to 5 C-atoms (i.e. 1,2,3,4,or 5 C-atoms) or n represents a number from 0 to 2. In a further particular embodiment the invention relates to a process as outlined above wherein R4 represents a saturated linear hydrocarbon with 1 to 5 C-atoms (i.e. 1,2,3,4,or 5 C-atoms) and n represents a number from 0 to 2.

Suitable methyl ketones for the process according to the invention include acetone, methyl ethyl ketone, methyl propyl ketone, methyl isopropyl ketone, methyl n-butyl ketone, methyl isobutyl ketone, methyl-tert.-butyl ketone without being limited thereto

Exemplary aldehydes for the process include 3-methylbutyraldehyde, 3-methyl crotonic aldehyde, citral, 3,7-dimethyl-2-octenal or 3,7-dimethyl-l-octanal without being limited thereto.

In a further preferred embodiment the invention relates to a process according to the invention and with the definitions and preferences as given above wherein the aldehyde of formula (5) is citral.

Furthermore, the process according to the invention is particulary suitable for carrying out the aldol condensation citral (formula (3) n = 1) with acetone (formula (4) R4 = methyl) to give ψ-ionone respectively with 2-pentanone (formula (3) R4 = propyl) to give 8,12-dimethyl-5,7,11-tridecatrien-4-one as depicted in the scheme below:

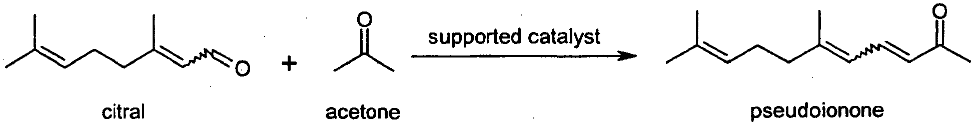

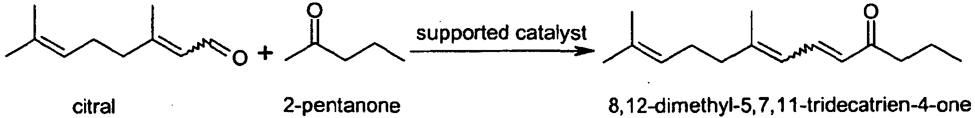

ψ-Ionone as well as 8,12-dimethyl-5,7,11-tridecatrien-4-one are important intermediates for vitamin and fragrance production.

A particular advantage of the inventive process is the use of the supported catalyst which exhibits an exceptional stability and allows simple regeneration by washing with organic solvents. Thus, the supported catalyst can be re-used while maintaining its activity. Preferably, in all embodiments of the invention the organic solvent used for washing of the supported catalyst is selected from acetone.

The preparation of supported catalysts is well known to a person skilled in the art. For example, the basic silica supported catalyst used in the process according to the invention may be obtained e.g. as described in WO01/87812 by impregnating a silica support with an aqueous solution of a calcium compound, optionally and preferably in the presence of a sodium compound followed by drying and calcinations if necessary to effect decomposition to the respective basic compounds. As calcium and sodium compound any compound which are basic or decompose to a basic compound upon heating such as for example calcium/ sodium hydroxide, acetate, oxalate, nitrate or carbonate may be used. As silica support any silica may be used such as e.g. silica support based on natural raw material or fumed pyrogenic silica which is e.g. available as Aerosil^{®} / Aerolyst^{®} from Degussa AG, Hanau, Germany. In all embodiments of the invention, the Ca/ Na on silica catalyst preferably has a metal loading of about 15 to 35 wt.-% Ca and about 20 to 35 wt.-% Na based on the total weight of the catalyst, more preferably a metal loading of about 20 to 30 wt.-% Ca and about 24 to 32 wt.-% Na.

The invention is not limited with respect to the shape and size of the usable catalyst particles; however, to improve the yield high specific surface areas and thus small catalyst particles are preferred. Suitable dimensions of the catalyst particles are, for example for solid cylindrical catalyst particles, from about 1*4 mm to about 4*40 mm.

The catalyst may be used as such or it may be suspended prior to its use. In a preferred embodiment of the invention the supported catalyst is added to the reaction mixture in pure solid form without further activation or modification. After termination of the reaction the catalyst can be recycled by simple technical measures such as filtration or decantation.

The amount of the supported catalyst used in the processes according to the invention is based on the amount of aldehyde. Usually the amount of the supported catalyst is in the range of 5 to 30wt%, preferably from 10 to 20 wt.-%, in particular in the range of 18 wt.-% based on the aldehyde. Such amounts of the supported catalyst are sufficient to obtain high yields of desired product.

The process according to the invention can be carried out without an additional solvent or in the presence of an additional solvent. Suitable solvents for the aldol condensation reaction are non-polar aprotic solvents, e.g. toluene, xylene, or ethers, e.g. diethyl ether methyl t-butyl ether. Preferably, in all embodiments of the invention the reaction is carried out without the addition of an additional solvent.

The ratio of ketone to aldehyde is not critical for the reaction and can vary over a wide range, although the ketone is normally used as excess component in order to achieve high product selectivity in relation to the aldehyde. Good results are obtained if a molar ratio of the aldehyde to the ketone of 1:0.5 to 1: 50, preferably 1:1 to 1:30, most preferably in the range of 1:10 to 1:3, in particular in the range of 1:4.4 is used in all embodiments of the invention. Advantageously, an excess of the ketone is used in the processes according to the invention.

The reaction can be performed at various temperatures and pressures the optimum being defined by the starting materials used. Generally, in all embodiments of the invention the reaction temperatures may vary from -20 to 200°C. Preferably, in all embodiments of the invention the reaction temperature is selected in the range of 20 to 100°C, in particular in the range of 30 to 60°C. The pressures may vary from 0.1 to 100 bar absolute. Preferably, in all embodiments of the invention the reaction is carried out at atmospheric pressure.

The process according to the invention can in principle be carried out in any reactor suitable for heterogeneously catalyzed reactions. Without restricting generality, the following are mentioned by way of example: suspension reactor, stirred tank, stirred tank cascade, tubular reactor, shell-type reactor, shell and tube reactor, fixed-bed reactor, fluidized-bed reactor, reactive distillation column.

In another embodiment, the invention relates to the use of Ca/Na on silica with the definitions and preferences as given above as catalyst for aldol condensation reactions. In particular the invention relates to the use of Ca/Na on silica with the definitions and preferences as given above as catalyst for aldol condensation reactions for the preparation of α,β-unsaturated ketones in particular pseudo ionone or 8,12-dimethyl-5,7,11-tridecatrien-4-one.

The invention is illustrated further by the examples without being limited thereto

### Example 1

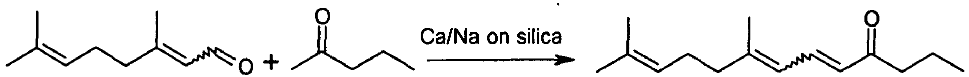

Citral (2.0 g, 95.0%, 12.5 mmol) and 2-pentanone (20 g, 99.0%, 229.9 mmol) were stirred under argon in a 2-neck-flask at 80 °C. Afterwards, Ca/Na on silica (360 mg, 18 wt% on citral) was added. The mixture was stirred for 4 h at 80 °C. After cooling to room temperature the mixture was filtered, the heterogeneous catalyst washed with 2-pentanone and all volatiles of the liquid phase were removed in vacuo (20 mbar, 40 °C). Yield: 87.5% (3.81 g, 63.3%, determined by GC, E/Z-mixture).

'H-NMR (300 MHz, CDCl₃): δ = 7.47 (dd, *J* = 11.5 Hz, 14.2 Hz, HC(6)); 7.44 (dd, *J* = 11.5 Hz, 15.2 Hz, HC(6)); 6.11 (d, *J* = 15.3 Hz, HC(5) or HC(7)); 6.07 (d, *J* = 15.2 Hz, HC(5) or HC(7)); 5.99 (d, *J* = 11.4 Hz, HC(5) or HC(7)); 5.12 (m, HC(11)); 2.53 (t, *J* = 7.3 Hz, H₂C(3)or H₂C(9)); 2.52 (t, *J* = 7.2 Hz, H₂C(3) or H₂C(9)); 2.32 (t, *J* = 7.9 Hz, H₂C(3) or H₂C(9)); 2.16 (s, CH₃), 1.90 (s, CH₃), 1.68 (m, CH₂, H₂C(2) and H₂C(10)); 1.61 (s, CH₃), 0.95 (t, *J* = 7.4 Hz, H₃C(1)); ¹³C-NMR (75 MHz, CDCl₃): δ = 201.0 (CO), 150.9 (C=C), 150.9 (C=C), 138.6 (C=C), 138.4 (C=C), 132.6 (C=C), 132.2 (C=C), 127.6 (C=C), 127.4 (C=C), 124.7 (C=C), 123.8 (C=C), 123.3 (C=C), 123.2 (C=C), 42.8, 42.7, 40.4, 33.0, 26.9, 26.3, 25.7, 24.6, 18.0, 17.9, 17.7, 17.5, 13.9.

### Example 2: Synthesis of Pseudoinone

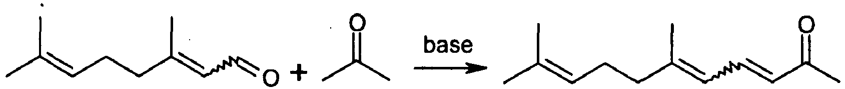

In a typical procedure for the aldol condensation citral (6.00 g, 91.4%, 36.02 mmol) and acetone (9.28 g, 99.5 %, 158.98 mmol) were kept under argon in a 2-neck-flask. The catalyst was added and the reaction mixture stirred at 57 °C and for 2.5 h. After cooling to room temperature, the reaction mixture was filtered and the residue once washed with acetone after. All volatiles of the liquid phase were removed in vacuo (20 mbar, 40 °C). Yield: 61.1% (8.42 g, 50.3%, determined by GC, E/Z-mixture).

### Example 3: Stability of the Ca/Na on silica compared to other heterogeneous aldolization catalysts.

The experiment of example 2 has been repeated several times, while recycling the respective catalysts as listed in the table below. As can be seen from table 1, the Ca/Na on silica catalyst does not loose its activity even after 10 turns whereas hydrotalcite or a basic ion exchange show a significant decrease in there activity already after the second turn.

**Table 1**

| Turn | Ambersep 900 OH | | Hydrotalcite, 3:1, 0.8 mol% NaOH dot. | | Hydrotalcite, 3:1, calc., 0.8 mol% NaOH dot. | | Hydrotalcite, 3:1, 0.8 mol% NaOH dot., calc. | | Ca/Na on silica | |
|---|---|---|---|---|---|---|---|---|---|---|
| | Y [%] | X [%] | Y [%] | X [%] | Y [%] | X [%] | Y [%] | X [%] | Y [%] | X [%] |
| 1 | 73.9 | 89.7 | 25.9 | 49.6 | 76.0 | 100.0 | 75.3 | 99.8 | 61.1 | 95.5 |
| 2 | 74.8 | 95.0 | 7.4 | 28.9 | 29.6 | 52.3 | 48.7 | 72.4 | 63.4 | 99.8 |
| 3 | 8.9 | 53.1 | 1.5 | 14.4 | 4.6 | 25.1 | 27.7 | 52.7 | 64.2 | 99.7 |
| 4 | 4.1 | 54.2 | 0.3 | 7.8 | 0.4 | 13.8 | 8.1 | 29.4 | 65.8 | 99.6 |
| 5 | | | | | | | | | 64.4 | 99.6 |
| 6 | | | | | | | | | 65.6 | 99.6 |
| 7 | | | | | | | | | 64.3 | 99.6 |
| 8 | | | | | | | | | 65.5 | 99.7 |
| 9 | | | | | | | | | 64.5 | 100.0 |
| 10 | | | | | | | | | 64.5 | 99.7 |

| | | | | | | | | | | |
|---|---|---|---|---|---|---|---|---|---|---|
| Y [%] = isolated yield X [%] = conversion determined by GC and a standard | | | | | | | | | | |

## Claims

1. A process for the production of α,β-unsaturated ketones, the process comprising the step of reacting an aldehyde of formula (1) 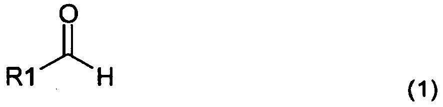 with a ketone of formula (2) 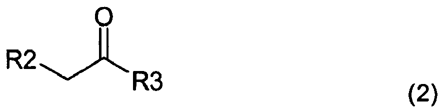 wherein R1 to R3 represent independently of each other a saturated or unsaturated, linear or branched hydrocarbons residue with 1 to 20 C-atoms or a cycloalkyl group with 4 to 12 C-atoms, which optionally may be substituted e.g. by one or several methoxy groups
in the presence of Ca/Na on silica as aldolization catalyst.

2. The process according to claim 1 for the production of α,β-unsaturated ketones of formula (3) 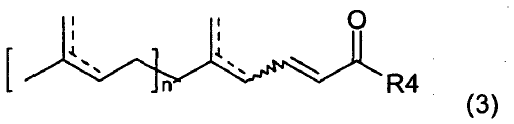 the process comprising reacting a methylketone of formula (4) 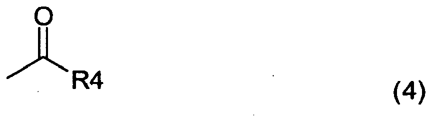 and an aldehyde of formula (5) 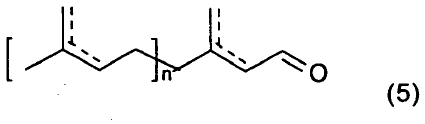 wherein
R4 represents a saturated or unsaturated, linear or branched hydrocarbons residue with 1 to 20 C-atoms and
n represents a number from 0 to 4 and
the dotted lines indicate, independently of each other, either a saturated or a double bond.

3. The process according to claim 2 wherein R4 is a linear, saturated hydrocarbon residue with 1 to 5 C-atoms.

4. The process according to claim 2 or 3, wherein n represent a number from 1 to 2.

5. The process according to anyone of claim 2 to 4, wherein the aldehyde of formula (5) is citral.

6. The process according to any of claims 1 to 5, wherein the Ca/Na on silica aldolization catalyst has a metal loading of 15 to 35 wt.-% Ca and 20 to 35 wt.-% Na based on the total weight of the catalyst, preferably a metal loading of 20 to 30 wt.-% Ca and 24 to 32 wt.-% Na.

7. The process according to anyone of claims 1 to 6, wherein the amount of catalyst is in the range of 18 wt.% based on Citral.

8. The process according to anyone of claim 1 to 7, wherein the molar ratio of the aldehyde to the ketone is in the range of 1:0.5 to 1: 50, preferably 1:1 to 1:30, most preferably in the range of 1:10 to 1:3.

9. Use of Ca/Na on silica as supported catalyst for aldol condensation reactions for the preparation of α,β-unsaturated ketones.

10. Use according to claim 9, wherein the Ca/Na on silica catalyst has a metal loading of 15 to 35 wt.-% Ca and 20 to 35 wt.-% Na based on the total weight of the catalyst, preferably a metal loading of 20 to 30 wt.-% Ca and 24 to 32 wt.-% Na.

11. Use according to claim 9 and 10 wherein the α,β-unsaturated ketones is pseudo ionone or 8,12-dimethyl-5,7,11-tridecatrien-4-one.

12. Use according claims 1-11, wherein the catalyst is Ca/Na on silica with a surface area of < 500 m²/g.

13. Use according claims 1-11, wherein the catalyst is Ca/Na on silica with a surface area of < 200 m²/g.

## Patentansprüche

1. Verfahren zur Herstellung von α,β-ungesättigten Ketonen, bei dem man einen Aldehyd der Formel (1) 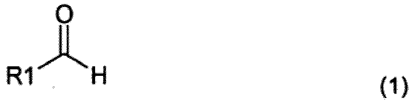 in Gegenwart von Ca/Na auf Siliciumdioxid als Aldolisierungskatalysator mit einem Keton der Formel (2) 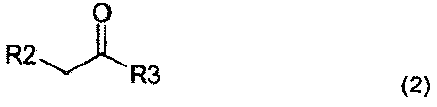 worin R1 bis R3 unabhängig voneinander für einen gesättigten oder ungesättigten, linearen oder verzweigten Kohlenwasserstoffrest mit 1 bis 20 C-Atomen oder eine Cycloalkylgruppe mit 4 bis 12 C-Atomen, die gegebenenfalls z.B. durch eine oder mehrere Methoxygruppen substituiert sein kann, stehen, umsetzt.

2. Verfahren nach Anspruch 1 zur Herstellung von α,β-ungesättigten Ketonen der Formel (3) 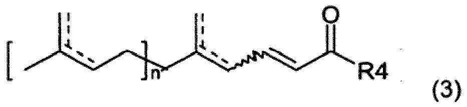 bei dem man ein Methylketon der Formel (4) 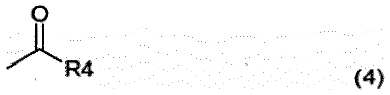 und einen Aldehyd der Formel (5) 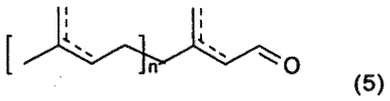 worin
R4 für einen gesättigten oder ungesättigten, linearen oder verzweigten Kohlenwasserstoffrest mit 1 bis 20 C-Atomen steht und
n für eine Zahl von 0 bis 4 steht und
die gestrichelten Linien unabhängig voneinander entweder eine gesättigte Bindung oder eine Doppelbindung andeuten, umsetzt.

3. Verfahren nach Anspruch 2, bei dem R4 für einen linearen, gesättigten Kohlenwasserstoffrest mit 1 bis 5 C-Atomen steht.

4. Verfahren nach Anspruch 2 oder 3, bei dem n für eine Zahl von 1 bis 2 steht.

5. Verfahren nach einem der Ansprüche 2 bis 4, bei dem es sich bei dem Aldehyd der Formel (5) um Citral handelt.

6. Verfahren nach einem der Ansprüche 1 bis 5, bei dem das als Aldolisierungskatalysator dienende Ca/Na auf Siliciumdioxid eine Metallbeladung von 15 bis 35 Gew.-% Ca und 20 bis 35 Gew.-% Na, bezogen auf das Gesamtgewicht des Katalysators, und vorzugsweise eine Metallbeladung von 20 bis 30 Gew.-% Ca und 24 bis 32 Gew.-% Na aufweist.

7. Verfahren nach einem der Ansprüche 1 bis 6, bei dem die Katalysatormenge im Bereich von 18 Gew.-%, bezogen auf Citral, liegt.

8. Verfahren nach einem der Ansprüche 1 bis 7, bei dem das Molverhältnis von Aldehyd zu Keton im Bereich von 1:0,5 bis 1:50, vorzugsweise 1:1 bis 1:30 und ganz besonders bevorzugt im Bereich von 1:10 bis 1:3 liegt.

9. Verwendung von Ca/Na auf Siliciumdioxid als Trägerkatalysator für Aldolkondensationsreaktionen zur Herstellung von α,β-ungesättigten Ketonen.

10. Verwendung nach Anspruch 9, wobei das als Katalysator dienende Ca/Na auf Siliciumdioxid eine Metallbeladung von 15 bis 35 Gew.-% Ca und 20 bis 35 Gew.-% Na, bezogen auf das Gesamtgewicht des Katalysators, und vorzugsweise eine Metallbeladung von 20 bis 30 Gew.-% Ca und 24 bis 32 Gew.-% Na aufweist.

11. Verwendung nach Anspruch 9 und 10, wobei es sich bei den α,β-ungesättigten Ketonen um Pseudoionon oder 8,12-Dimethyl-5,7,11-tridecatrien-4-on handelt.

12. Verwendung nach den Ansprüchen 1-11, wobei es sich bei dem Katalysator um Ca/Na auf Siliciumdioxid mit einer Oberfläche < 500 m²/g handelt.

13. Verwendung nach den Ansprüchen 1-11, wobei es sich bei dem Katalysator um Ca/Na auf Siliciumdioxid mit einer Oberfläche < 200 m²/g handelt.

## Revendications

1. Procédé de production de cétones α,β-insaturées, le procédé comprenant l'étape consistant à réagir un aldéhyde de formule (1) 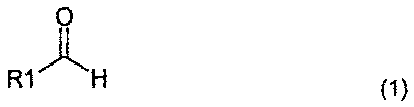 avec une cétone de formule (2) 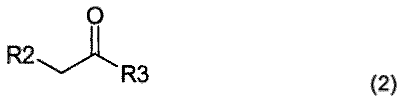 dans lesquelles R1 à R3 représentent, indépendamment les uns des autres, un reste hydrocarboné linéaire ou ramifié, saturé ou insaturé, ayant de 1 à 20 atomes de C ou un groupement cycloalkyle ayant de 4 à 12 atomes de C, pouvant être éventuellement substitué par exemple par un ou plusieurs groupements méthoxy,
en présence de Ca/Na sur silice comme catalyseur d'aldolisation.

2. Procédé selon la revendication 1, pour la production de cétones α,β-insaturées de formule (3) 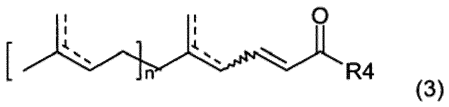 le procédé comprenant la réaction d'une méthylcétone de formule (4) 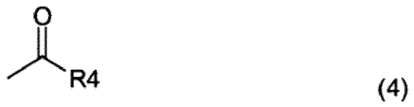 et d'un aldéhyde de formule (5) 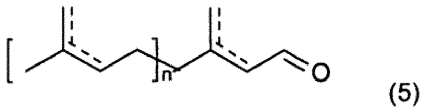 dans lesquelles
R4 représente un reste hydrocarboné linéaire ou ramifié, saturé ou insaturé, ayant de 1 à 20 atomes de C, et
n représente un nombre allant de 0 à 4, et
les lignes pointillées indiquent, indépendamment les unes des autres, une liaison saturée ou bien double.

3. Procédé selon la revendication 2, dans lequel R4 représente un reste hydrocarboné linéaire saturé ayant de 1 à 5 atomes de C.

4. Procédé selon la revendication 2 ou 3, dans lequel n représente un nombre allant de 1 à 2.

5. Procédé selon l'une quelconque des revendications 2 à 4, dans lequel l'aldéhyde de formule (5) est le citral.

6. Procédé selon l'une quelconque des revendications 1 à 5, dans lequel le catalyseur d'aldolisation à base de Ca/Na sur silice possède une charge en métal allant de 15 à 35% en poids de Ca et de 20 à 35% en poids de Na sur la base du poids total du catalyseur, préférablement une charge en métal allant de 20 à 30% en poids de Ca et de 24 à 32% en poids de Na.

7. Procédé selon l'une quelconque des revendications 1 à 6, dans lequel la quantité de catalyseur se trouve dans la plage de 18% en poids sur la base du citral.

8. Procédé selon l'une quelconque des revendications 1 à 7, dans lequel le rapport molaire de l'aldéhyde à la cétone se trouve dans la plage allant de 1:0,5 à 1:50, préférablement de 1:1 à 1:30, tout préférablement dans la plage allant de 1:10 à 1:3.

9. Utilisation de Ca/Na sur silice comme catalyseur sur support pour les réactions de condensation d'aldol destinées à la préparation de cétones α,β-insaturées.

10. Utilisation selon la revendication 9, dans laquelle le catalyseur à base de Ca/Na sur silice possède une charge en métal allant de 15 à 35% en poids de Ca et de 20 à 35% en poids de Na sur la base du poids total du catalyseur, préférablement une charge en métal allant de 20 à 30% en poids de Ca et de 24 à 32% en poids de Na.

11. Utilisation selon les revendications 9 et 10, dans laquelle les cétones α,β-insaturées sont la pseudoionone ou la 8,12-diméthyl-5,7,11-tridécatrièn-4-one.

12. Utilisation selon les revendications 1-11, dans laquelle le catalyseur est du Ca/Na sur silice ayant une surface < 500 m²/g.

13. Utilisation selon les revendications 1-11, dans laquelle le catalyseur est du Ca/Na sur silice ayant une surface < 200 m²/g.
